# EUROPEAN PATENT SPECIFICATION

(11) **EP 1 067 899 B1**
(45) Date of publication and mention of the grant of the patent: **11.01.2012**
(21) Application number: 99917832.0
(22) Date of filing: 22.03.1999
(51) Int. Cl.: A61K 8/39, A61K 8/41, A61K 8/893, A61K 8/898, A61Q 5/12

(54) **HAIR CONDITIONING COMPOSITIONS**
HAARPFLEGEMITTEL
COMPOSITIONS CAPILLAIRES

(30) Priority: 30.03.1998 GB 9806826
(43) Date of publication of application: 17.01.2001
(73) Proprietor: Unilever PLC, London EC4Y 0DY (GB); Unilever N.V., 3013 AL Rotterdam (NL)
(72) Inventor: MURRAY, Andrew, Malcolm, Wirral Merseyside L63 3JW (GB)
(74) Representative: Newbould, Frazer Anthony
(86) International application number: PCT/EP1999/001902
(87) International publication number: WO 1999/049836

(56) References cited:
- EP-A- 0 152 194
- GB-A- 2 157 168
- GB-A- 2 316 615
- US-A- 4 586 518
- M.S. STARCH: "Silicones in Hair Care Products" DRUG AND COSMETIC INDUSTRY, vol. 134, no. 6, 1906 - 1984, XP002109041

## Description

### FIELD OF THE INVENTION

This invention relates to hair conditioning compositions intended to be rinsed off. In particular, the invention relates to hair conditioning compositions containing emulsified particles of amino functional silicone, which compositions condition the hair leaving it softer and more manageable.

### BACKGROUND AND PRIOR ART

The use of silicones as conditioning agents in cosmetic formulations is well known and widely documented in the patent literature. Hair treatment compositions containing amino functional polysiloxanes have also been described in several publications. For example, U.S. 4,563,347 teaches that an aqueous emulsion of aminoalkyl substituted polydimethylsiloxane is useful to condition hair because it facilitates combing and imparts a smooth feel to hair. Other hair treating compositions containing amino functional polysiloxanes are described in U.S. 4,586,518, U.S. 4,601,902 and U.S. 4,618,819. Amino functional microemulsions have also been described in the field of hair care. For example, U.S. 4,749,732 describes hair care uses of polydiorganosiloxanes containing aminoalkyl groups modified by alkoxycarbonylalkyl substituents. U.S. 4,620,878 describes generally the preparation of emulsions of silicones containing polar substituents, and teaches a method of preparing clear microemulsions of amine functional polyorganosiloxanes, which can be mixed with a shampoo base of sodium lauryl ether sulphate and water to produce a stable, clear composition.

GB2316615 discloses hair conditioning compositions comprising conditioning cationic surfactants, emulsified particles of amino-functional silicone such as amodimethicone and non-aminofunctionalised silicones.

A problem encountered with hair conditioning formulations incorporating amino functional silicones is that the delivery of conditioning performance may be insufficient for many people, particularly in regions such as Japan and South East Asia where consumers desire a high level of conditioning and a "weighty" feel to their hair.

We have now found that the inclusion in a hair conditioning composition of a particular class of amino functional silicone, with specified mole percent amino functionality, significantly improves wet and dry conditioning performance.

### SUMMARY OF THE INVENTION

The invention provides an aqueous hair conditioning composition according to claim 1.

### DETAILED DESCRIPTION OF THE INVENTION

### Conditioning Surfactant

The composition according to the invention comprises one or more conditioning surfactants which are cosmetically acceptable and suitable for topical application to the hair.

Suitable conditioning surfactants are selected from cationic surfactants, used singly or in admixture. Examples include quaternary ammonium hydroxides or salts thereof, e.g chlorides.

Suitable cationic surfactants for use in hair conditioning compositions of the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, and the corresponding hydroxides thereof. Further suitable cationic surfactants include those materials having the CTFA designations Quaternium-5, Quaternium-31 and Quaternium-18. Mixtures of any of the foregoing materials may also be suitable. Particularly useful cationic surfactant for use in hair conditioners of the invention are cetyltrimethylammonium chloride and behenyltrimethylammonium chloride

In hair conditioning compositions of the invention, the level of cationic surfactant is preferably from 0.01 to 10%, more preferably 0.05 to 5%, most preferably 0.1 to 2% by weight based on total weight of the composition.

### Emulsified amino functionalised silicone

Hair conditioning compositions according to the invention include emulsified particles of an amino functionalised silicone of general formula:

Si(CH₃)₃ - O - [Si(CH₃)₂ - O - ]ₓ - [Si(CH₃) (R - NH-CH₂CH₂ NH₂) - O -]_{y} - Si (CH₃)₃

wherein x + y is a number from about 50 to about 500, and wherein R is an alkylene group having from 2 to 5 carbon atoms.

Preferably, the number x + y is in the range of from about 100 to about 300.

The amino functional silicone is insoluble in the aqueous matrix of the hair conditioning composition and so is present in an emulsified form, with the silicone present as dispersed particles.

We have found that amino functional silicones suitable for use in the invention need to have a mole percent amino functionality of at least 1.0 mole %. Suitably the mole percent amino functionality of the amino functional silicone ranges from about 1 to about 8.0 mole %, preferably from about 1 to about 5.0 mole %, such as about 1.7 mole %.

Various methods of making emulsions of particles of amino functional silicones for use in the invention are available and are well known and documented in the art. For example, emulsions may be prepared by high shear mechanical mixing of the silicone and water, or by emulsifying the silicone with water and an emulsifier (mixing the silicone into a heated solution of the emulsifier for instance), or by a combination of mechanical and chemical emulsification. A further suitable technique for preparation of emulsions of particles of silicones is emulsion polymerisation. Emulsion polymerised silicones as such are described in US 2 891 820 (Hyde), US 3 294 725 (Findlay) and US 3 360 491 (Axon).

The viscosity of the amino functional silicone itself (not the emulsion or the final hair conditioning composition) is not particularly critical and can suitably range from about 50 to 500,000 cst. Viscosity can be measured by means of a glass capillary viscometer as set out further in Dow Corning Corporate Test Method CTM004 July 20 1970.

The average silicone particle size of the emulsified particles of amino functional silicone in the hair conditioning composition composition is less than 20, preferably less than 10 microns. We have found that reducing the particle size generally improves conditioning performance. Most preferably the average amino functional silicone particle size in the hair conditioning composition is less than 2 microns, ideally it ranges from 0.01 to 1 micron. Silicone emulsions having an average silicone particle size of ≤ 0.15 microns are generally termed microemulsions.

Particle size may be measured by means of a laser light scattering technique, using a 2600D Particle Sizer from Malvern Instruments.

Specific examples of amino functional silicones suitable for use in the invention are the aminosilicone oils DC2-8220, DC2-8166, DC2-8466, and DC2-8950-114 (all ex Dow Corning), and GE 1149-75, (ex General Electric Silicones).

Also suitable are pre-formed emulsions of amino functional silicone oils with non ionic and/or cationic surfactant. This is particularly preferred since the pre-formed emulsion can be incorporated into the hair conditioning composition by simple mixing.

Pre-formed emulsions of amino functionalised silicone are available from suppliers of silicone oils such as Dow Corning, General Electric, Union Carbide, Wacker Chemie, Shin Etsu, Toshiba, Toyo Beauty Co, and Toray Silicone Co. Examples include emulsions DC2-8320, DC2-8306, DC2-8177 and DC2-8467, all available from Dow Corning.

### - Emulsified, non-amino functionalised silicone

Hair conditioning compositions of the invention also comprise emulsified particles of a non-amino functionalised silicone, which is insoluble in the aqueous matrix of the composition and so is present in an emulsified form, with the silicone present as dispersed particles.

Suitable non-amino functionalised silicones include polydiorganosiloxanes, in particular polydimethylsiloxanes which have the CTFA designation dimethicone. Also suitable for use in hair conditioning compositions of the invention are polydimethyl siloxanes having hydroxyl end groups, which have the CTFA designation dimethiconol. Also suitable for use in hair conditioning compositions of the invention are silicone gums having a slight degree of cross-linking, as are described for example in WO 96/31188. These materials can impart body, volume and stylability to hair, as well as good wet and dry conditioning.

The viscosity of the emulsified non-amino functionalised silicone itself (not the emulsion or the final hair conditioning composition) is typically at least 10,000 cst. In general we have found that conditioning performance increases with increased viscosity. Accordingly, the viscosity of the silicone itself is preferably at least 60,000 cst, most preferably at least 500,000 cst, ideally at least 1,000,000 cst. Preferably the viscosity does not exceed 10⁹ cst for ease of formulation.

Emulsified non-amino functional silicones for use in hair conditioning compositions of the invention will typically have an average silicone particle size in the composition of less than 2 microns, ideally it ranges from 0.01 to 1 micron.

Suitable non-amino functional silicone emulsions for use in the invention are also commercially available in a pre-emulsified form.

Examples of suitable pre-formed emulsions include emulsions DC2-1766, DC2-1784, and microemulsions DC2-1865 and DC2-1870, all available from Dow Corning. These are all emulsions/microemulsions of dimethiconol. Cross-linked silicone gums are also available in a pre-emulsified form, which is advantageous for ease of formulation. A preferred example is the material available from Dow Corning as DC X2-1787, which is an emulsion of cross-linked dimethiconol gum. A further preferred example is the material available from Dow Corning as DC X2-1391, which is a microemulsion of cross-linked dimethiconol gum.

The total amount of silicone (amino functional and non-amino functional, if present) incorporated into the hair conditioning compositions of the invention depends on the level of conditioning desired and the material used. A preferred amount is from 0.01 to about 10% by weight of the total composition although these limits are not absolute. The lower limit is determined by the minimum level to achieve conditioning and the upper limit by the maximum level to avoid making the hair and/or skin unacceptably greasy.

We have found that a total amount of silicone of from 0.3 to 5%, preferably 0.5 to 3%, by weight of the total composition is a suitable level.

### - Optional Ingredient

### - Fatty Alcohol

Hair conditioning compositions of the invention advantageously incorporate a fatty alcohol material. The combined use of fatty alcohol materials and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 20. Examples of suitable fatty alcohols include cetyl alcohol, stearyl alcohol and mixtures thereof. The use of these materials is also advantageous in that they contribute to the overall conditioning properties of compositions of the invention.

Alkoxylated, (e.g. ethoxylated or propoxylated) fatty alcohols having from about 12 to about 18 carbon atoms in the alkyl chain can be used in place of, or in addition to, the fatty alcohols themselves. Suitable examples include ethylene glycol cetyl ether, polyoxyethylene (2) stearyl ether, polyoxyethylene (24) cetyl ether, and mixtures thereof.

The level of fatty alcohol and/or alkoxylated fatty alcohol material in conditioners of the invention is conveniently from 0.01 to 10%, preferably from 0.1 to 5% by weight of the composition.

The weight ratio of cationic surfactant to fatty alcohol and/or alkoxylated fatty alcohol is suitably from 10:1 to 1:10, preferably from 4:1 to 1:8, optimally from 1:1 to 1:4.

### - Other optional ingredients

Compositions of this invention may contain any other ingredient normally used in hair treatment formulations. These other ingredients may include viscosity modifiers, preservatives, colouring agents, polyols such as glycerine and polypropylene glycol, chelating agents such as EDTA, antioxidants, fragrances, and sunscreens. Each of these ingredients will be present in an amount effective to accomplish its purpose. Generally these optional ingredients are included individually at a level of up to about 5% by weight of the total composition.

Preferably, compositions of this invention also contain adjuvants suitable for hair care. Generally such ingredients are included individually at a level of up to 2%, preferably up to 1%, by weight of the total composition.

Among suitable hair care adjuvants, are:
(i) natural hair root nutrients, such as amino acids and sugars. Examples of suitable amino acids include arginine, cysteine, glutamine, glutamic acid, isoleucine, leucine, methionine, serine and valine, and/or precursors and derivatives thereof. The amino acids may be added singly, in mixtures, or in the form of peptides, e.g. di- and tripeptides. The amino acids may also be added in the form of a protein hydrolysate, such as a keratin or collagen hydrolysate. Suitable sugars are glucose, dextrose and fructose. These may be added singly or in the form of, e.g. fruit extracts. A particularly preferred combination of natural hair root nutrients for inclusion in compositions of the invention is isoleucine and glucose. A particularly preferred amino acid nutrient is arginine.
(ii) hair fibre benefit agents. Examples are:
   - ceramides, for moisturising the fibre and maintaining cuticle integrity. Ceramides are available by extraction from natural sources, or as synthetic ceramides and pseudoceramides. A preferred ceramide is Ceramide II, ex Quest. Mixtures of ceramides may also be suitable, such as Ceramides LS, ex Laboratoires Serobiologiques.
   - fatty acids, for cuticle repair and damage prevention. Examples are branched chain fatty acids such as 18-methyleicosanoic acid and other homologues of this series, straight chain fatty acids such as stearic, myristic and palmitic acids, and unsaturated fatty acids such as oleic acid, linoleic acid, linolenic acid and arachidonic acid. A preferred fatty acid is oleic acid. The fatty acids may be added singly, as mixtures, or in the form of blends derived from extracts of, e.g. lanolin.

Mixtures of any of the above active ingredients may also be used. A particularly preferred combination is arginine and oleic acid.

The invention is further illustrated by way of the following non-limitative Example:

### EXAMPLE

A hair conditioning composition was prepared by mixing the following components in the amounts stated:

### Ingredient

| | Example | Reference Example | Example |
|---|---|---|---|
| | % wt | | |
| Arquad 16-50 | 1.4 | 1.4 | 1.4 |
| Laurex CS | 1.8 | 5.0 | 3.1 |
| Paraffin wax | -- | -- | 1.0 |
| Silicone DC-1766 | 2.5 | -- | 2.216 |
| Silicone DC-8177 | 3.1 | 10.0 | 2.063 |
| Polysurf 67 | 0.02 | -- | -- |
| Preservative | < | qs | > |
| Perfume | 0.3 | 0.3 | 0.3 |

| | | | |
|---|---|---|---|
| Arquad 16-50 is a 50% solution of cetyltrimethylammonium chloride in isopropanol Laurex CS is a mixture of cetyl and stearyl alcohol Silicone DC-1766 is a 60% emulsion of dimethiconol Silicone DC-8177 is a 15% emulsion of trimethylsilylamodimethicone with a particle size of 40nm Polysurf 67 is cetyl hydroxyethylcellulose | | | |

## Claims

1. An aqueous hair conditioning composition comprising, in addition to water:
(i) at least one cationic surfactant, and
(ii) emulsified particles of an amino functional silicone of general formula:
Si(CH₃)₃ - O - [Si(CH₃)₂ - O - ]ₓ - [Si(CH₃) (R - NH-CH₂CH₂ NH₂)- O -]_{y} - Si(CH3)₃
wherein x + y is a number from 50 to 500, and wherein R is an alkylene group having from 2 to 5 carbon atoms;
in which the amino functional silicone has a mole percent amino functionality of at least 1 mole % and which further comprises emulsified particles of a non-amino functionalised silicone and wherein the average particle size of the amino functionalised silicone is less than 20 microns and the average particle size of the non-amino functionalised silicone is less than 2 microns.

2. A composition according to claim 1, in which the mole percent amino functionality of the amino functional silicone ranges from 1 to about 8.0 mole %.

3. A composition according to claim 1 or claim 2, in which the average amino functional silicone particle size in the hair conditioning composition is less than 2 micrometres (microns).

4. A composition according to claim 1, in which the viscosity of the emulsified non-amino functionalised silicone itself is at least 60,000 cst.

5. A composition according to any preceding claim, which further comprises a fatty alcohol and/or an alkoxylated fatty alcohol.

6. A composition according to claim 5, in which the weight ratio of cationic surfactant to fatty alcohol and/or alkoxylated fatty alcohol is from 1:1 to 1:4.

## Patentansprüche

1. Wässrige Haarkonditionierungs-Zusammensetzung, umfassend zusätzlich zu Wasser:
(i) wenigstens ein kationisches Tensid und
(ii) emulgierte Partikel eines aminofunktionalisierten Silikons der allgemeinen Formel:
Si(CH₃)₃- O - [Si(CH₃)₂ - O]ₓ - [Si (CH₃) (R - NH - CH₂CH₂-NH₂) - O - ]y - Si (CH₃)₃
worin x + y eine Zahl von 50 bis 500 ist und worin R eine Alkylen-Gruppe mit 2 bis 5 Kohlenstoffatomen ist;
wobei das aminofunktionalisierte Silikon eine Aminofunktionalität in Molprozent von wenigstens 1 Mol-% hat, und die außerdem emulgierte Partikel eines nicht-aminofunktionalisierten Silikons umfasst und wobei die durchschnittliche Partikelgröße des aminofunktionalisierten Silikons weniger als 20 Mikrometer ist und die durchschnittliche Partikelgröße des nicht-aminofunktionalisierten Silikons weniger als 2 Mikrometer (Mikron) ist.

2. Zusammensetzung gemäß Anspruch 1, wobei die Aminofunktionalität in Molprozent des aminofunktionellen Silikons von etwa 1 bis etwa 8,0 Mol-% reicht.

3. Zusammensetzung gemäß Anspruch 1 oder Anspruch 2, wobei die durchschnittliche Partikelgröße des aminofunktionellen Silikons in der Haarkonditionierungs-Zusammensetzung weniger als 2 Mikrometer (Mikron) ist.

4. Zusammensetzung gemäß Anspruch 1, in der die Viskosität des emulgierten nicht-aminofunktionalisierten Silikons selbst wenigstens 60 000 cSt ist.

5. Zusammensetzung gemäß einem vorangehenden Anspruch, die außerdem einen Fettalkohol und/oder einen alkoxylierten Fettalkohol umfasst.

6. Zusammensetzung gemäß Anspruch 5, in der das Gewichtsverhältnis von kationischem Tensid zu Fettalkohol und/oder alkoxyliertem Fettalkohol von 1:1 bis 1:4 ist.

## Revendications

1. Composition capillaire aqueuse comprenant, en plus de l'eau :
(i) au moins un agent tensio-actif cationique, et
(ii)des particules émulsifiées d'une silicone amino-fonctionnelle de formule générale :
Si(CH₃)₃-O-[Si(CH₃)₃)₂-O-]ₓ-[Si(CH₃)(R-NH-CH₂CH₂NH₂)-O-]_{y}-Si(CH₃)₃
oùx+y est un nombre de 50 à 500, et où R est un groupe alkylène ayant de 2 à 5 atomes de carbone ;
dans laquelle la silicone amino-fonctionnelle a une amino-fonctionnalité en pourcentage molaire d'au moins 1 % molaire et qui comprend en outre des particules émulsifiées d'une silicone non-amino-fonctionnalisée et où la taille de particule moyenne de la silicone amino-fonctionnalisée est de moins de 20 microns et la taille de particule moyenne de la silicone non-amino-fonctionnalisée est de moins de 2 microns.

2. Composition selon la revendication 1, dans laquelle l'amino-fonctionnalité en pourcentage molaire de la silicone amino-fonctionnelle vari de 1 à environ 8,0 % molaire.

3. Composition selon la revendication 1 ou la revendication 2, dans laquelle la taille de particule moyenne de la silicone amino-fonctionnelle dans la composition capillaire est de moins de 2 micromètres (microns).

4. Composition selon la revendication 1, dans laquelle la viscosité de la silicone non-amino-fonctionnalisée émulsifiée, elle-même est d'au moins 60 000 cst.

5. Composition selon l'une quelconque des revendications précédentes, qui comprend en outre un alcool gras et/ou un alcool gras alcoxylé.

6. Composition selon la revendication 5, dans laquelle le rapport de poids entre l'agent tensio-actif cationique et l'alcool gras et/ou l'alcool gras alcoxylé est de 1:1 à 1:4.
